# EUROPÄISCHE PATENTSCHRIFT

(11) **EP 3 642 347 B1**
(45) Veröffentlichungstag und Bekanntmachung des Hinweises auf die Patenterteilung: **07.06.2023**
(21) Anmeldenummer: 17737733.0
(22) Anmeldetag: 23.06.2017
(51) Int. Cl.: C12P 7/40, C12P 7/42, C12P 7/44, C12P 7/48, C12N 9/88

(54) **VERFAHREN ZUR HERSTELLUNG VON CARBONSÄUREN UNTER UNSTERILEN BEDINGUNGEN**
METHOD FOR PRODUCING CARBOXYLIC ACIDS UNDER UNSTERILE CONDITIONS
PROCÉDÉ DE PRÉPARATION D'ACIDES CARBOXYLIQUES DANS DES CONDITIONS NON STÉRILES

(43) Veröffentlichungstag der Anmeldung: 29.04.2020
(73) Patentinhaber: Helmholtz-Zentrum für Umweltforschung GmbH-UFZ, 04318 Leipzig (DE)
(72) Erfinder: AURICH, Andreas, 04207 Leipzig (DE); HUNGER, Steffi, 04827 Machern (DE); BECKER, Mi-Young, 33615 Bielefeld (DE); KOHLHEB, Norbert, 1149 Budapest (HU); MÜLLER, Roland Arno, 04420 Markranstädt (DE)
(74) Vertreter: Gulde & Partner
(86) Internationale Anmeldenummer: PCT/EP2017/065589
(87) Internationale Veröffentlichungsnummer: WO 2018/233851

(56) Entgegenhaltungen:
- SVETLANA V KAMZOLOVA ET AL: "Lipase Secretion and Citric Acid Production in Yarrowia lipolytica Yeast Grown on Animal and Vegetable Fat", FOOD TECHNOLOGY AND BIOTECHNOLOGY, Bd. 43, Nr. 2, 12. September 2004 (2004-09-12), Seiten 113-122, XP055454931, Croatia ISSN: 1330-9862
- MASANORI WATANABE ET AL: "Fermentative l-(+)-lactic acid production from non-sterilized rice washing drainage containing rice bran by a newly isolated lactic acid bacteria without any additions of nutrients", JOURNAL OF BIOSCIENCE AND BIOENGINEERING, Bd. 115, Nr. 4, April 2013 (2013-04), Seiten 449-452, XP055454814, NL ISSN: 1389-1723, DOI: 10.1016/j.jbiosc.2012.11.001
- ZHANYOU CHI ET AL: "Lipid Production by Culturing Oleaginous Yeast and Algae with Food Waste and Municipal Wastewater in an Integrated Process", APPLIED BIOCHEMISTRY AND BIOTECHNOLOGY ; PART A: ENZYME ENGINEERING AND BIOTECHNOLOGY, HUMANA PRESS INC, NEW YORK, Bd. 165, Nr. 2, 13. Mai 2011 (2011-05-13), Seiten 442-453, XP019956385, ISSN: 1559-0291, DOI: 10.1007/S12010-011-9263-6
- FABIO SANTAMAURO ET AL: "Low-cost lipid production by an oleaginous yeast cultured in non-sterile conditions using model waste resources", BIOTECHNOLOGY FOR BIOFUELS, BIOMED CENTRAL LTD, GB, Bd. 7, Nr. 34, 4. März 2014 (2014-03-04), Seiten 1-11, XP021179076, ISSN: 1754-6834, DOI: 10.1186/1754-6834-7-34
- MESUT TASKIN ET AL: "Microbial lipid production by cold-adapted oleaginous yeast Yarrowia lipolytica B9 in non-sterile whey medium", BIOFUELS, BIOPRODUCTS & BIOREFINING, Bd. 9, Nr. 5, 17. April 2015 (2015-04-17), Seiten 595-605, XP055454890, GB ISSN: 1932-104X, DOI: 10.1002/bbb.1560
- DIMITRIS SARRIS ET AL: "Production of added-value metabolites by Yarrowia lipolytica growing in olive mill wastewater-based media under aseptic and non-aseptic conditions", ENGINEERING IN LIFE SCIENCES, Bd. 17, Nr. 6, 13. März 2017 (2017-03-13), Seiten 695-709, XP055454899, DE ISSN: 1618-0240, DOI: 10.1002/elsc.201600225

## Beschreibung

Die Erfindung betrifft ein Verfahren zur biotechnologischen Herstellung von Carbonsäuren.

Bekanntermaßen können durch die Kultivierung von Bakterien, Hefen und filamentösen Pilzen eine Vielzahl von Mono-, Di- und Tricarbonsäuren wie Äpfel-, Bernstein-, Brenztrauben-, Citronen-, Fumar-, Glucon-, Itacon-, Ketoglutarsäure oder Milchsäure in hohen Produktkonzentrationen, Produktbildungsraten, Selektivitäten und Ausbeuten gewonnen werden. Diese Carbonsäuren stehen für eine Vielzahl von Anwendungen in diversen Industriezweigen (z.B. Chemie, Lebensmittel-, Pharmabereich) zur Verfügung.

Als Kohlenstoffquellen für Wachstum und die Carbonsäurebildung der Mikroorganismen werden dabei überwiegend Kohlenhydrate, Proteine und Lipide aus nachwachsenden Rohstoffen sowie Abprodukte eingesetzt, welche diese Substratklassen enthalten. Die Verwendung von Kohlenstoffquellen aus landwirtschaftlichen Anbau für biotechnologische Verfahren steht dabei häufig in Konkurrenz zu deren Einsatz als Lebens- und Futtermittel.

Um hohe Produktivitätskennziffern zu erzielen, werden die biotechnologischen Produktionsverfahren für Carbonsäuren nahezu ausschließlich unter Einhaltung allgemein bekannter steriler (aseptischer) Kultivierungsbedingungen durchgeführt, wobei insbesondere die Kohlenstoffquellen, Nährmedienbestandteile, die erforderliche Luftzufuhr und das Reaktorequipment für die Durchführung der Bioproduktionsstufe entsprechenden Aufwendungen (z.B. durch chemische oder thermische Sterilisation, Filter) zur Erzeugung und Aufrechterhaltung der Keimfreiheit unterzogen werden.

Die überwiegend als Submerskultivierungen ausgeführten biotechnologischen Produktionsverfahren verlangen die Bereitstellung von erheblichen Mengen frischem Brauchwasser, häufig in Trinkwasserqualität und diese überschreitenden Qualitäten. Nach Durchführung der Kultivierungen und Abtrennung der Biomassen und Carbonsäuren muss das verbleibende Prozesswasser gewöhnlich einer konventionellen Abwasserbehandlung zugeführt werden.

Eine herausragende Stellung bezüglich der biotechnologisch erzeugten Carbonsäuren nimmt die Citronensäure (2-Hydroxypropan-1,2,3-tricarbonsäure) mit einer weltweiten Jahresproduktion von 1,6 Millionen Tonnen ein. Aufgrund ihrer geschmacklichen, antioxidativen, farbstabilisierenden und komplexbildenden Eigenschaften findet Citronensäure bekanntermaßen eine breite Anwendung in der chemischen Industrie (z.B. Wasch- und Reinigungsmittelzusatz, Entkalker), Lebensmittel- und Getränkeindustrie (z.B. Geschmacksverbesserung) sowie pharmazeutischen Industrie (z.B. Stabilisierung von Blutkonserven).

Als industrielle Produktionsverfahren für Citronensäure sind seit Jahrzehnten Submersverfahren mit dem filamentösen Pilz *Aspergillus niger* etabliert. Dabei werden ausschließlich Kohlenhydrate wie Saccharose, Glucose, Stärkehydrolysate und kohlenhydrathaltige Abprodukte, insbesondere Zuckerrohr- und Rübenmelassen, als Kohlenstoffquellen eingesetzt.

Trotz jahrzehntelanger Optimierung des Aspergillus-Prozesses besitzt dieser eine Reihe von Nachteilen. Insbesondere nachteilig bei allen Submersverfahren zur Citratgewinnung ist die erforderliche vollständige sterile Prozessführung, in welche die Sterilisierung (110-130°C für 0,5-1 h) oder Pasteurisierung (85-95°C) der Kohlenstoffquellen und Sterilfiltration der Luft für die Sauerstoffversorgung der Pilzkulturen eingeschlossen ist. Dafür sind erhebliche energetische und apparative Aufwendungen vorzusehen.

Ebenso nachteilig sind bei der submersen Citratherstellung mit *Aspergillus niger* die hohen Anforderungen bezüglich der Reinheit des Betriebswassers für die Kultivierung. Es sollte mindestens Trinkwasserqualität eingesetzt werden. Zusätzlich erfordert die geringe Toleranz von *Aspergillus niger* gegenüber Schwermetallen deren vorherige Ausfällung aus den eingesetzten Substraten und Prozesswässern durch Zusatz von Cyaniden (z.B. Hexacyanoferrat) vor der Bioprozessstufe.

Hefebasierte Verfahren zur Gewinnung von Citronensäure wurden und werden als Alternative zu dem *Aspergillus* niger-Verfahren seit den 60er Jahren des vorigen Jahrhunderts entwickelt.

Vorrangig wurde dabei die Hefeart *Yarrowia lipolytica* eingesetzt, die sich durch eine hohe Vielfalt an verwertbaren Kohlenstoffquellen für die Citronensäurebildung auszeichnet und damit den Nachteil des engen nutzbaren Substratspektrums, welches *Aspergillus niger* kennzeichnet, überwindet.

Die Kultivierungen mit *Y. lipolytica* erfolgen im Allgemeinen unter vollständig sterilen Bedingungen (inklusive steriler Kohlenstoffquellen und Belüftung) und werden bevorzugt im Batch-, Fedbatch-, oder semikontinuierlichen Betrieb realisiert.

Nachteilig für alle gentechnisch veränderten *Y. lipolytica* Hefestämme ist, dass deren Einsatz den Bestimmungen des deutschen Gentechnikgesetztes (GenTG) aber auch vergleichbaren internationalen Regelungen unterliegt, und damit explizit eine sterile Prozessführung in geschlossenen Systemen erfordert, welche ein Austreten der GVO in die Umwelt verhindert. Hefebasierte Verfahren zur Citratbildung mit *Y. lipolytcia* werden, vergleichbar mit den *A. niger* Verfahren, gewöhnlich unter Einsatz eines Betriebswassers mit mindestens Trinkwasserqualität durchgeführt.

D. Sarris et al., ENGINEERING IN LIFE SCIENCES, 2017, Vol. 17, Nr. 6, Seiten 695 - 709, lehrt die fermentative Produktion von Citronensäure mit der Hefe Yarrowia lipolytica unter Verwendung von Ölmühlenabwasser in einem Glucose enthaltenden Nährmedium. Es findet immer eine Vorbehandlung statt, einschließlich Pasteurisierung des Kulturmediums bei 80°C für 5min.

S. Kamzolova et al., Food Technology and Biotechnology, 2004, Vol. 43, Nr. 2, Seiten 113-12, lehrt die Produktion von Lipiden und Citronensäure mit Yarrowia lipolytica Hefen unter Verwendung von reinen tierischen oder pflanzlichen Fetten als Kohlenstoffquelle.

M. Taskin et al., BIOFUELS, BIOPRODUCTS & BIOREFINING, 2015, Vol. 9, No. 5, Seiten 595-605, lehrt die fermentative Produktion von Fettsäuren mit Yarrowia lipolytica in einem Wasser, Molke und Lactose enthaltenden Medium. Die Molke wird bei bei 90°C thermisch behandelt, was einer Pasteurisierung entspricht.

Die der Erfindung zugrunde liegende Aufgabe besteht darin, die Nachteile der bestehenden Verfahren zur biotechnologischen Herstellung von Carbonsäuren zu vermeiden und solche Verfahren bereitzustellen, welche hohe Produktkonzentrationen und Produktivitäten bei gleichzeitiger Schonung der Ressourcen Wasser und Energie ermöglicht.

Die Lösung der Aufgabe erfolgt mit der Erfindung gemäß Anspruch 1, die Unteransprüche sind Vorzugsvarianten.

Erfindungsgemäß ist ein submerses Verfahren zur biotechnologischen Herstellung von Carbonsäuren mit Mikroorganismen Stämmen der Hefearten Yarrowia lipolytica vorgesehen, wobei die Mikroorganismen unsteril in einem Abwasser unter Zugabe von kohlenstoffreichen Verbindungen kultiviert werden.

Der Begriff "Carbonsäuren" in der Erfindung bezeichnet die Intermediate des Tricarbonsäurezyklus: Aconit-, Äpfel-, Bernstein-, Citronen-, Fumar-, Isocitronen-, α-Ketoglutar-, Oxalessigsäure, sowie die mit dem Tricarbonsäurezyklus assoziierten Verbindungen Brenztrauben- und Itaconsäure. Besonders bevorzugt ist Citronensäure und deren Salze (z.B. Calzium-, Kalium- und Natriumcitrate).

Der Begriff "Mikroorganismen" umfasst die Hefeart *Yarrowia lipolytica.* Ganz besonders bevorzugt wird der Stamm *Yarrowia lipolytica* H181 (DSM 7806) verwendet, welcher bei der Deutschen Sammlung von Mikroorganismen und Zellkulturen GmbH unter der Nummer DSM 7806 hinterlegt ist.

Mit dem Begriff "Abwasser" werden alle Wässer bezeichnet, die durch ihre Nutzung und Gebrauch verunreinigt sind. Bevorzugt wird der Einsatz von Grauwasser, industriellen (z.B. aus der Lebensmittelindustrie, chemischer, pharmazeutischer und biotechnologischer Industrie, Erdgas- und Erdölgewinnung und -verarbeitung) und kommunalen Abwässern vorgesehen. Besonders bevorzugt werden Abwässer aus der Lebensmittelverarbeitung (z.B. Reinigungswässer des Küchen- und Cateringbetriebes) und kommunale Abwässer nach deren Vorklärung vorgesehen.

Unter dem Begriff "kohlenstoffreiche Verbindung", wie hier verwendet, werden organische kohlenstoffhaltige Substrate verstanden, die von Mikroorganismen als heterotrophe Kohlenstoffquelle utilisiert werden. Die kohlenstoffreichen Verbindungen umfassen Mono-, Di-, Triglyceride, Fettsäuren, Fettsäuremethyl- oder ethylester, Ethanol, Methanol, Glycerol, Glucose, Fruktose, Galactose, Lactose, Milchsäure, Saccharose, Xylose, Arabinose und/oder n-Alkane.

Diese kohlenstoffhaltigen Verbindungen werden durch landwirtschaftliche Produktion gewonnen oder stellen überwiegende biogene Abprodukte dar. Der Anteil des utilisierbaren Kohlenstoffanteils ist dabei vom Reinheits- und Aufbereitungsgrad der Substrate abhängig. Bevorzugt werden kohlenstoffreiche Verbindungen aus landwirtschaftlicher Produktion oder in Form von Abprodukten eingesetzt, welche Mono-, Di-, Triglyceride, Fettsäuren, Fettsäuremethyl- oder ethylester, Ethanol, Methanol, Glycerol, Glucose, Fruktose, Galactose, Lactose, Milchsäure, Saccharose, Xylose, Arabinose und/oder n-Alkane (n-Paraffin) enthalten.

Besonders bevorzugt wird die Verwendung von Abprodukten in Form von genutzten Frittierölen (Altfrittenöl), welche Mono-, Di-, Triglyceride und Fettsäuren enthalten, vorgesehen.

Die eingesetzten kohlenstoffreichen Verbindungen können dabei schon partiell oder vollständig in dem Abwasser bei dessen Anfallen vorliegen oder diese werden vollständig dem Abwasser zugefügt. Ebenso können Gemische von zwei oder mehreren kohlenstoffreichen Verbindungen für die Erfindung genutzt werden.

Mit dem Begriff "unsteril" wird bezeichnet, dass vor, während und nach der Kultvierung keine üblichen, bekannten Maßnahmen (z.B. chemische oder thermische Sterilisierung, Pasteurisierung) und Vorrichtungen (z.B. Sterilfilter für Belüftung, sterile Probenentnahme) zur Erzeugung und zum Erhalt einer Keimfreiheit und Sterilität und Vermeidung des Eindringens von Fremdkeimen in das Kultivierungsmedium vorgesehen werden.

Anhand der hefebasierten biotechnologischen Gewinnung von besonders bevorzugt produzierter Citronensäure wird die Erfindung detailliert beschrieben, ohne sie darauf zu beschränken.

Als bevorzugte Hefegattungen mit der Befähigung, Citronensäure in hohen Konzentrationen zu akkumulieren, haben sich Mikroorganismen Stämme der Hefearten Yarrowia lipolytica erwiesen. Insbesondere Mutanten, Selektanten und rekombinante Stämme der Hefeart *Yarrowia lipolytica* besitzen die Fähigkeit zur Citratproduktion mit einer Selektivität von größer 90%, wobei Isocitronensäure als einziges signifikantes Nebenprodukt auftritt.

Erfindungsgemäß werden vorzugsweise *Yarrowia lipolytica* Stämme, bevorzugt *Y. lipolytica* H181 (DSM 7806), unter unsterilen aeroben, submersen Bedingungen in einem abwasserhaltigen Kultvierungsmedium, welches die üblichen, bekannten synthetischen oder halbsynthetischen Nährmedienbestandteile und Kohlenstoffquellen für die Bildung und Ausscheidung von Citronensäure enthält, kultiviert. Dabei wird die Bildung und Akkumulation der Citronensäure in bekannter Weise durch Limitation einer Nährstoffkomponente (z.B. Stickstoff, Phosphor oder Schwefel), vorzugsweise Ammoniumstickstoff, ausgelöst. Dem Kultivierungsmedium wird eine für die Citratbildung erforderliche stammspezifische Thiaminkonzentration von >0,5 mg/L Thiamin × HCl, vorzugsweise 1 mg/L und bis zu 1000 mg/L zugegeben. Die Kultivierung wird erfindungsgemäß bevorzugt in der Art und Weise ausgeführt, dass in einer Reaktionseinrichtung ein Abwasser in unsteriler Form vorgelegt wird.

Als Reaktionseinrichtungen werden vorzugsweise alle Reaktionsbehälter vorgesehen, die für eine Submerskultivierung geeignet sind, sich aber hinsichtlich ihrer Bauart, Ausführung, Art der Mischung und Belüftung unterscheiden können. Als Reaktionsbehälter sind z.B. übliche Rührreaktoren, Airliftreaktoren, Blasensäulen aber auch abwasserbehandlungsrelevante Ausführungen wie Belebungsbecken oder Sequencing batch-Reaktoren zu benennen. Die Reaktionseinrichtungen können in geschlossener oder offener Bauweise ausgeführt sein. Offene Bauweise bedeutet, dass ein Eindringen von Mikroorganismen aus der Umwelt in die Reaktionseinrichtung möglich ist.

Anschließend werden dem vorgelegten Abwasser alle erforderlichen Nährkomponenten und kohlenstoffreichen Verbindungen unsteril zugeführt. Abschließend wird dem Reaktionsbehälter die Animpfkultur (Inokulum) der Hefe zugeführt, welche über eine oder mehrere Stufen auf übliche, bekannte Art erzeugt wird. Die Vorkulturstufen können dabei vollständig oder partiell unsteril ausgeführt werden.

Für die Erfindung können prinzipiell alle Arten von Abwasser eingesetzt werden, welche das Wachstum der Hefe *Yarrowia lipolytica* nicht inhibieren. Bevorzugt ist die Verwendung von Abwasser aus den Reinigungsstufen des Küchen- und Cateringsbetriebes, Abläufen von gewerblichen Öl-/Fettseparatoren und der Vorklärungsstufe von Abwasserbehandlungsanlagen für kommunale Abwässer. Die Abwässer werden dem Reaktionsbehälter in einem Volumenanteil von 5-100% des gesamten Kultivierungsmediums zugeführt; vorzugsweise mit einem Anteil von 90-100%.

Als kohlenstoffreiche Verbindungen (Kohlenstoffquellen) können im Prinzip alle Verbindungen für die Erfindung verwendet werden, welche von *Yarrowia lipolytica* verstoffwechselt werden können, und ausgewählt sind aus Mono-, Di-, Triglyceriden, Fettsäuren, Fettsäuremethyl- oder ethylestern, Ethanol, Methanol, Glycerol, Glucose, Fruktose, Galactose, Lactose, Milchsäure, Saccharose, Xylose, Arabinose und/oder n-Alkanen.

Die Kohlenstoffquellen können als Einzelverbindungen oder Mischungen eingesetzt werden.

Bevorzugt ist der Einsatz von benutzen pflanzlichen oder tierischen Fetten oder Ölen, besonders bevorzugt von Altfrittenöl.

Die Kohlenstoffquelle wird nach bekannten Kultivierungsregimes entweder bereits zu Beginn der Kultivierung (Batch-Betrieb) vollständig zugeführt oder es erfolgt zu bestimmten Zeitpunkten eine Nachdosierung des Substrates (Fedbatch-Betrieb).

Vorzugweise werden die Altfrittenöle mit einer Konzentration 35-40 g/L im Reaktionsbehälter vorgelegt und unsteril mit einem üblichen Feeding-Regime auf Endkonzentrationen von 100-180 g/L nachdosiert.

Zusätzlich ist bevorzugt, dass die Kultivierung von *Y. lipolytica* in üblicher Batch-, Fedbatch-, Repeated Fedbatch (semikontinuierlicher) oder kontinuierlicher Betriebsweise erfolgt, wobei der C-Betrieb besonders bevorzugt ist.

Die Kultivierung von *Y. lipolytica* erfolgt bei einem pH-Wert von 2,0 bis 8,0 und einer Temperatur von 5 bis 45°C, vorzugsweise bei pH 3,5 bis 5,5 und einer Temperatur von 26 bis 33°C. Als Neutralisationsmittel für die pH-Statierung sind bevorzugt CaCO₃, Ca(OH)₂, KOH, NaOH, Na₂CO₃, NaHCO₃ geeignet, besonders bevorzugt NaOH.

Die Citratbildung von *Y. lipolytica* als bekanntermaßen aerober Bioprozess erfordert die Zufuhr von Sauerstoff während der Kultivierung, wobei die Sauerstoffverteilung im Kultivierungsmedium wie üblich durch Belüftung und Umwälzung realisiert wird. Die Gelöstsauerstoffkonzentration, gemessen als pO₂-Wert, sollte in einem Bereich von pO₂=1% bis 100% liegen, vorzugsweise ist eine Sauerstoffsättigung von pO₂ ≥ 20% zu gewährleisten.

Die Kultivierung von *Y. lipolytica* beginnt mit einer Wachstumsphase, welche in der Regel mit Auszehrung der wachstumslimitierenden Nährstoffkomponente nach 8 bis 30 h in die Produktbildungsphase für Citronensäure übergeht. Üblicherweise wird die Kultivierung zu einem ökonomischen Zeitpunkt bezüglich der Produktivität z.B. bei erheblich abnehmender oder stagnierender Carbonsäurebildung abgebrochen. Die Gesamtkultivierungszeit dauert im Allgemeinen etwa 60 bis 250 Stunden in Abhängigkeit von der eingesetzten Menge der Kohlenstoffquelle und dem gewählten Kultivierungs-Modus (z.B. Fedbatch- und Repeated Fedbatch).

Vorteilhafterweise ermöglicht das erfindungsgemäße Verfahren Citronensäurekonzentrationen von ≤210 g/L.

Die erfindungsgemäß gewonnene Kulturlösung, welche die produzierte Citronensäure und Hefebiomasse von *Y. lipolytica* enthält, kann anschließend den für die Produktisolierung und -konzentrierung von Carbonsäuren üblichen Verfahren des Downstream processing zugeführt werden. Diese Verfahren beinhalten mindesten eine übliche Abtrennstufe für Mikroorganismen (z.B. Crossflow-Filtration, Sedimentation, Filtration, Zentrifugation) und eine Isolierstufe für Citronensäure (z.B. Adsorption/Desorption, Ausfällung, Elektrodialyse mit bipolaren Membranen, Simulated Moving Bed (SMB)-Chromatografie, Reaktivextraktion).

Die Anwendung des erfindungsgemäßen Verfahrens ist mit deutlichen Vorteilen im Vergleich zum gegenwärtigen Stand der Technik verbunden, die wie folgt zusammengefasst werden können:
- Produktkonzentrationen von ≤ 210 g/L Citronensäure liegen über mit den bekannten Verfahren erzielten Ausbeuten.
- Die Produktion von Citronensäure unter komplett unsterilen Bedingungen vermeidet die apparativen, energetischen und materiellen Aufwendungen, die bei den bestehenden Verfahren für die Erzeugung und Aufrechterhaltung von sterilen Kulturbedingungen erforderlich sind.
- Die vorgesehene ausschließliche Nutzung von Abwässern für die submerse Kultivierung vermeidet die bei den bestehenden Verfahren vorgesehene Nutzung von Betriebswasser und höheren Qualitäten sowie die apparativen, energetischen und materiellen Aufwendungen für deren Herstellung.
- Der bevorzugte Einsatz von Abprodukten als kohlenstoffreiche Verbindungen für die Produktion von Citronensäure vermeidet die konkurrierende Nutzung von Kohlenstoffquellen aus dem landwirtschaftlichen Anbau, welche als Lebens- und Futtermittel eingesetzt werden können.
- Die Produktionskulturen können als freie Hefezellen eingesetzt werden und damit Aufwendungen für eine Zellimmobilisierung, wie sie teilweise in den beschriebenen Verfahren und Veröffentlichungen erforderlich sind, vermieden werden.

In den nachfolgenden Beispielen wird die Erfindung näher beschrieben.

### Beispiel 1

### Gewinnung Vorkultur

Der Stamm *Yarrowia lipolytica* H181 (DSM 7806) wurde auf einem bekannten für Hefen geeigneten Nährmedium (z.B. Pepton Hefeextrakt Agar oder Reader-Agar) für 24 bis 48 h bei 30°C kultiviert.

Von der gewachsenen Agarkultur wurden 2 bis 3 Impfösen auf 100 ml einer sterilen mit destilliertem Wasser hergestellten Vorkulturlösung folgender Zusammensetzung überimpft:

| | |
|---|---|
| NH₄Cl | 3,00 g/L |
| KH₂PO₄ | 0,70 g/L |
| MgSO₄ x 7 H2O | 0,35 g/L |
| NaCl | 0,10 g/L |
| CaCl₂ x 2 H₂O | 0,13 g/L |
| FeSO₄ x 7 H₂O | 3,5 mg/L |
| Thiamin x HCl | 1 mg/L |
| Spurensalzlösung | 5 ml/L |
| Sonnenblumenöl | 50 g/L |

Die Spurensalzlösung des Vorkulturmediums hatte folgende Zusammensetzung:

| | |
|---|---|
| CuSO₄ x 5 H₂O | 4,0 g/L |
| MnSO₄ x 5 H₂O | 4,0 g/L |
| ZnCl₂ | 2,1 g/L |
| CoSO₄ x 7 H₂O | 0,5 g/L |
| H₃BO₃ | 5,7 g/L |

Die Vorkultur wurde und bei pH 5-6 in 500 ml-Erlenmeyerkolben mit Schikanen bei 30 °C für 48 h auf einer Schüttelapparatur bei einer Schüttelfrequenz von 120 bis 140 rpm kultiviert.

### Durchführung Haupt-/Produktionskultur

Von der Vorkultur erfolgte die Überimpfung von 30 ml Inokulum bei einem Animpfverhältnis 1:10 in einen 0,5 L-Rührreaktor mit 300 ml Arbeitsvolumen zur Durchführung der unsterilen submersen Haupt-/Produktionskultivierung, wobei die Begriffe Haupt- und Produktionskultur als Synonyme verwendet werden. Das Produktionsmedium enthielt folgende Komponenten:

| | |
|---|---|
| NH₄Cl | 3,00 g/L |
| KH₂PO₄ | 0,70 g/L |
| MgSO₄ x 7 H₂O | 0,35 g/L |
| CaCl₂ x 2 H₂O | 0,13 g/L |
| FeSO4 x 7 H2O | 3,5 mg/L |
| Thiamin x HCl | 1 mg/L |
| Spurensalzlösung | 1 ml/l |

### (Zusammensetzung der Spurensalzlösung wie Vorkultur)

Als Kohlenstoffquelle enthielt das Produktionsmedium ein Altfrittenöl, welches zuvor für zwei Tage als Frittieröl zur Herstellung von Pommes frites verwendet wurde. Das Altfrittenöl wies folgendes Fettsäuremuster (Masseprozente) auf:

| | |
|---|---|
| Myristinsäure (14:0) | 5,4% |
| Palmitinsäure (16:0) | 0,2% |
| Stearinsäure (18:0) | 1,6% |
| Ölsäure (18:1; w9c) | 58,7% |
| Vaccensäure (18:1; w7c) | 3,5% |
| Linolsäure (18:2) | 19,6% |
| Linolensäure (18:3) | 8,7% |
| Arachdonsäure (20:0) | 0,4% |
| Eicosensäure (20:1; w9c) | 0,9% |
| andere Fettsäuren | 1,0% |

Als flüssige Phase für die Herstellung des Produktionsmediums wurde das Abwasser aus dem Ablauf eines Öl- und Fettseparators eines gewerblichen Kantinen- und Cateringbetriebes eingesetzt. Der Ablauf des Öl-/Fettseparators war durch folgende abwasserbezogenen Kennziffern gekennzeichnet:

| Parameter | |
|---|---|
| pH | 6,3 |
| DOC | 6,7 mg O₂/L |
| Leitfähigkeit | 2,17 mS/cm |
| CSB | 2830 mg/L |
| BSB₅ | 1611 mg/L |
| TOC | 954 mg/L |
| TSS | 576 mg/L |
| TN | 32 mg/L |

Alle Nährmedienbestandteile des Produktionsmediums wurden unsteril in dem Abwasser gelöst und dem 0,5 L-Rührreaktor zugegeben. Die Startkonzentration des Altfrittenöls im Rührreaktor betrug 40 g/L und wurde unsteril im Fedbatch-Betrieb in Schritten von 20 g/L bis auf eine Endkonzentration von 140 g/L zugegeben. Die Produktionskulturen wurden als Doppelansatz bei 30°C, Rührerdrehzahl = 900 rpm und einer Gelöstsauerstoffkonzentration pO₂=50% durchgeführt. Die Gelöstsauerstoffkonzentration wurde über einen Gasmix aus Luft/Stickstoff/Sauerstoff geregelt, wobei die Belüftung der Rührreaktoren unsteril ohne Zu- und Abluftfilter mit einem Gesamtgasfluss von 0,5 L/min erfolgte. Der pH-Wert wurde bei pH 5 mit 20%iger NaOH statiert.

Zum Vergleich wurden sterile Doppelansätze, mit a.) destilliertem Wasser (inklusive aller Nährkomponenten sterilisiert bei 121°C für 20 min), anstelle Abwasser und Altfrittenöl (pasteurisiert bei 80°C für 20 min) sowie b.) Öl-/Fettseparatorablauf (inklusive aller Nährkomponenten, sterilisiert bei 121°C für 20 min) und Altfrittenöl (pasteurisiert bei 80°C für 20 min) realisiert.

Mit Auszehrung der Stickstoffquelle Ammoniumsulfat nach 21 h begann die Akkumulation von Citronensäure und Isocitronensäure in den Kulturlösungen. Nach 166 h wurden die Kultivierungen beendet. Für den unsterilen Ansatz mit Abwasser (Ablauf Öl-/Fettseparator) wurde eine Carbonsäurebildung von 137 g/L Citronensäure und 11 g/L des Nebenproduktes Isocitronensäure festgestellt (Mittelwerte aus zwei Kultivierungen). Dies entsprach einer Produktivität von 0,83 g/L*h. Bei Nutzung von Abwasser unter sterilen Bedingungen waren die Produktmengen an Carbonsäuren nur unwesentlich erhöht; mit destilliertem Wasser wurden die niedrigsten Konzentrationen an Citrat gebildet (Tabelle 1).

**Tab. 1 Citronensäureproduktion von Yarrowia lipolytica H181 (DSM 7806) unter unsterilen Kulturbedingungen (Mittelwerte aus je zwei identischen Versuchsansetzen)**

| Ansatz | Kultur- dauer | Citronen- säure (g/L) | Isocitronen- säure (g/L) | Produktivität Citrat (g/L*h) | Selektivität Citrat (%) |
|---|---|---|---|---|---|
| **unsteril** | | | | | |
| Ablauf Öl-Separator + Altfrittenöl | 166 h | 137 ± 8,5 | 11,2 ± 1,4 | 0,83 ± 0,05 | 92,4 ± 0,5 |

| **steril** | | | | | |
|---|---|---|---|---|---|
| a.) Destilliertes Was- ser + Altfrittenöl | 166 h | 125 ± 4,6 | 15,2 ± 3,7 | 0,75 ± 0,03 | 89,3 ± 2,0 |
| b.) Ablauf Öl- Separator + Altfrittenöl | 166 h | 145 ± 9,6 | 14,9 ± 0,6 | 0,88 ± 0,06 | 90,7 ± 0,2 |

### Beispiel 2

Die Kultivierung erfolgte, wie in Beispiel 1 beschrieben, wobei ein Abwasser (Ablauf Öl-/Fettseparator) in der Hauptkultur eingesetzt wurde, welches folgende Zusammensetzung hatte:

| Parameter | |
|---|---|
| pH | 5,9 |
| DOC | 1,7 mg O₂/L |
| Leitfähigkeit | 3,0 mS/cm |
| CSB | 1760 mg/L |
| BSB₅ | 1036 mg/L |
| TOC | 676 mg/L |
| TSS | 315 mg/L |
| TN | 29 mg/L |

Die Hauptkultur wurde als semikontinuierliche Kultivierung (repeated Fedbatch Betrieb) in einem 15-L-Rührreaktor durchgeführt. Dabei wurde Zyklus 1 mit 9 L Arbeitsvolumen gestartet, welches 900 ml der Vorkultur von *Y. lipolytica* H181 (DSM 7806) und eine initiale Altfrittenöl-Konzentration von 40 g/L enthielt. Das Feeding der Kohlenstoffquelle erfolgte bis zu einer summarischen Endkonzentration von 120 g/L Altöl.

Abweichend von Beispiel 1 wurde der pH-Wert 5 mit 45%iger NaOH statiert und die Sauerstoffversorgung der Hefen mittels einer Belüftungsmenge von 4 L/min bei einer Rührerdrehzahl von 800 rpm realisiert. Die Gelöstsauerstoffkonzentration betrug über die gesamte Kultivierung pO₂ ≥ 20%.

Mit Auszehrung der Stickstoffquelle Ammoniumsulfat nach 20 h konnte die Akkumulation von Citronensäure und Isocitronensäure registriert werden. Nach 70 h Kulturdauer wurde Zyklus 1 der Hauptkultivierung beendet. Das Volumen der Kulturlösung betrug 10,5 L und enthielt 77 g/L Citronensäure, 3,8 g/l Isocitronensäure. Dies entsprach einer Produktivität von 1,1 g/L*h und Selektivität von 95,2% für Citronensäure.

Nach Abschluss von Zyklus 1 wurden 9,5 L der Kulturlösung bis auf 1 L Restvolumen abgelassen und unsteril mit 8 L des Öl-/Fettseparatorablaufes inklusive der Nährmedienkomponenten des Produktionsmediums aufgefüllt. Die in dem Restvolumen von Zyklus 1 enthaltene Hefebiomasse von *Y. lipolytica* H181 fundierte als Inokulum für die Fortsetzung der Kultivierung im zweiten Kultivierungszyklus.

In Zyklus 2 wurden dem Rührreaktor 150 g/L Altfrittenöl als externe Kohlenstoffquelle im Fedbatch-Modus zudosiert. Bezüglich des pH-Wertes und der Sauerstoffversorgung wurden die Bedingungen von Zyklus 1 eingehalten. Mit wiederholter Auszehrung Stickstoffquelle Ammoniumsulfat nach 20 h in Zyklus 2 begann die Bildung von Citronensäure und Isocitronensäure durch die Hefe *Yarrowia lipolytica* H181. Bei Abbruch von Zyklus 2 nach 95 h Zyklusdauer, bei einer Gesamtkultvierungszeit von 165 h über beide Zyklen, betrug die Konzentration an Citronensäure 91 g/L und Isocitronensäure 4,4 g/L in 10,6 L Kulturlösung. Damit waren die Produktivität (0,96 g/L*h) und Selektivität (95,4%) der Citratbildung in Zyklus 2 nahezu identisch mit den Ergebnissen von Zyklus 1.

Insgesamt konnten in beiden unsterilen Zyklen 20,1 L Kulturlösung geerntet werden, welche 1,7 Kg Citronensäure enthielten.

### Beispiel 3

Die in Beispiel 2 beschriebene Kultivierung wurde dahingehend modifiziert, dass auch die Vorkultur unter Verwendung eines Abwassers (Ablauf Öl-/Fettseparator) gewonnen wurde. Zusätzlich abweichend von Beispiel 2 erfolgte die unsterile Hauptkultur mit einem unbehandelten Abwasser eines gewerblichen Kantinen- und Cateringbetriebes, welches als Zulauf in einen Öl-/Fettabscheider eingeleitet wird. Das verwendete Abwasser (Zulauf Öl-/Fettabscheider) hatte folgende Zusammensetzung:

| Parameter | |
|---|---|
| pH | 5,8 |
| DOC | 0,6 mg O₂/L |
| Leitfähigkeit | 2,9 mS/cm |
| CSB | 5240 mg/L |
| BSB₅ | 1661 mg/L |
| TOC | 1095 mg/L |
| TSS | 191 mg/L |
| TN | 117 mg/L |

Die Hauptkultur wurde als Fedbatch-Kultivierung in einem 15-L-Rührreaktor mit 9 L Startvolumen durchgeführt. Die initiale Altfrittenölkonzentration betrug 35 g/L und wurde durch kontinuierliche Substratzufuhr bis auf 175 g/L nach 120 h Kulturdauer erhöht. Abweichend von Beispiel 2 wurde die Rührerdrehzahl auf 600 rpm abgesenkt. Die Gelöstsauerstoffkonzentration betrug während der Kultivierung pO₂ ≥ 5%.

Nach 21 h war die Ammoniumstickstoffquelle ausgezehrt; ab diesem Zeitpunkt erfolgte die Akkumulation von Citrat und Isocitrat. Bei Abbruch der Hauptkultivierung nach 166 h konnten 181 g/L Citronensäure und 5,4 g/L Isocitronensäure in der Kulturbrühe gemessen werden. Dies einsprach einer Produktivität von 1,1 g/(L*h) für Citronensäure. Die Selektivität für Citrat betrug 97%.

### Beispiel 4

Die Kultivierung wurde wiederholt, wie in Beispiel 2 beschrieben, wobei ein kommunales Abwasser (Ablauf Septic tank) als flüssige Phase in der Hauptkultur verwendet wurde. Das kommunale Abwasser hatte folgende Zusammensetzung:

| Parameter | |
|---|---|
| pH | 7,2 |
| DOC | 0,76 mg O₂/L |
| Leitfähigkeit | 1,66 mS/cm |
| CSB | 486 mg/L |
| BSB₅ | 287 mg/L |
| TOC | 163 mg/L |
| TSS | 65 mg/L |
| TN | 91 mg/l |
| *E. coli* | 4,6 × 10⁶ MPN/1 00 mL |

Abweichend von Beispiel 2 wurde die unsterile Hauptkultur als Fedbatch-Kultivierung (15 L-Rührreaktor mit 9 L Arbeitsvolumen) durchgeführt. Dabei wurde Altfrittenöl als Kohlenstoffquelle bis zu einer Endkonzentration von 180 g/L zudosiert, ausgehend von einer initialen Substratkonzentration von 40 g/L. Die Kultivierung erfolgte bei pH 5, welcher mit 45%iger NaOH eingestellt wurde und einer Rührerdrehzahl von 600 rpm. Die Luftbegasungsrate betrug 4 L/min. Damit konnten über die gesamte Kulturdauer die Gelöstsauerstoffkonzentrationen pO₂ ≥ 3% gehalten werden.

Nach Auszehrung der NH4-N-Quelle bei 21 h wurden innerhalb der nachfolgenden von 56 h Kultivierung 95 g/L Citronensäure und 2 g/L Isocitrat von *Y. lipolytica* H181 (DSM 7806) gebildet, entsprechend einer Citronensäurebildungsrate von 1,2 g/(L*h). Die Fortsetzung der Kultvierung bis 189 h führte zu einer weitere Erhöhung der Citroenensäurekonzentration bis auf 133,7 g/L. Bei gleichzeitig gemessenen 4,8 g/L Isocitronensäure konnte eine Selektivität von 96,5% für die Citratbildung ermittelt werden. Die Produktivität für Citrat betrug für den Gesamtzeitraum 0,71 g/(L*h).

Bei Kultivierungsende waren in der Kulturlösung keine *E. coli*-Keime (Summenparameter pathogene Bakterien) mehr nachweisbar. Dies ist ein Indiz für hygienisierende Wirkung der hefebasierten Citratproduktion auf Abwässer.

### In den vorstehenden Beispielen 1 bis 4 werden folgende Abkürzungen verwendet:

- pH: pH-Wert
- DOC: Dissolved oxygen content
- CSB: Chemischer Sauerstoffbedarf
- BSB₅: Biologischer Sauerstoffbedarf (nach 5 Tagen)
- TOC: Total organic carbon
- TSS: Total suspended solids
- TN: Total nitrogen
- E. coli: Escherichia coli

## Patentansprüche

1. Verfahren zur biotechnologischen Herstellung von Carbonsäuren, bei dem Mikroorganismen eingesetzt werden, die unter unsterilen Bedingungen in einem Kultivierungsmedium, welches Abwasser enthält, das kohlenstoffreiche Verbindungen enthält und/oder dem kohlenstoffereiche Verbindungen zugegeben werden, kultiviert werden, wobei
Mikroorganismen Stämme der Hefeart *Yarrowia lipolytica* verwendet werden;
und wobei die herzustellenden Carbonsäuren Aconit-, Äpfel-, Bernstein-, Citronen-, Fumar-, Isocitronen-, α-Ketoglutar-, Oxalessig-, Brenztrauben- und/oder Itaconsäure, bevorzugt Citronensäure umfassen; und wobei
die kohlenstoffreiche Verbindungen Mono-, Di-, Triglyceride, Fettsäuren, Fettsäuremethyl- oder ethylester, Ethanol, Methanol, Glycerol, Glucose, Fruktose, Galactose, Lactose, Milchsäure, Saccharose, Xylose, Arabinose und/oder n-Alkane umfassen.

2. Verfahren nach Anspruch 1, **dadurch gekennzeichnet, dass** der Stamm *Yarrowia lipolytica* H181 (DSM 7806) eingesetzt wird.

3. Verfahren nach Anspruch 2, **dadurch gekennzeichnet, dass** der Stamm der *Yarrowia lipolytica* H181 (DSM 7806) unter unsterilen aeroben, submersen Bedingungen in einem abwasserhaltigen Kultvierungsmedium kultiviert wird.

4. Verfahren nach einem der vorherigen Ansprüche, **dadurch gekennzeichnet, dass** es sich bei dem eingesetzten Abwasser um ein Wasser handelt, welches durch Nutzung und Gebrauch verunreinigt ist, vorzugsweise ist das Abwasser ein Grauwasser, industrielles Abwasser aus der Lebensmittelindustrie, chemischer, pharmazeutischer und biotechnologischer Industrie, Pflanzenölverarbeitung, Erdgas- und Erdölgewinnung und -verarbeitung und/oder kommunales Abwasser und/oder aus den Reinigungsstufen eines Küchen- und/oder Cateringsbetriebes, Abläufen von gewerblichen Öl-/Fettseparatoren und/oder der Vorklärungsstufe von Abwasserbehandlungsanlagen für kommunale Abwässer.

5. Verfahren nach einem der vorherigen Ansprüche, **dadurch gekennzeichnet, dass** das Abwasser einem Reaktionsbehälter in einem Volumenanteil von 10-100% des gesamten Kultivierungsmediums zugeführt wird; vorzugsweise mit einem Anteil von 90-100%.

6. Verfahren nach einem der vorherigen Ansprüche, **dadurch gekennzeichnet, dass** als kohlenstoffreiche Verbindungen Abprodukte von benutzten pflanzlichen und/oder tierischen Fetten und/oder Ölen, vorzugsweise von Altfrittenöl eingesetzt werden, die die kohlenstoffreichen Verbindungen enthalten, oder dass Mischungen kohlenstoffreicher Verbindungen verwendet werden.

7. Verfahren nach einem der vorherigen Ansprüche, **dadurch gekennzeichnet, dass** die Kultivierung unsteril durchgeführt wird und keinerlei Maßnahmen und Vorrichtungen zur Erzeugung und zum Erhalt einer Keimfreiheit und Sterilität und Vermeidung des Eindringens von Fremdkeimen in das Kultivierungsmedium vorgesehen sind.

8. Verfahren nach einem der vorherigen Ansprüche, **dadurch gekennzeichnet, dass** die Kultivierung in einer Reaktionseinrichtung durchgeführt wird, die in offener oder geschlossener Bauweise oder Konstruktion vorliegt, vorzugsweise in Reaktionsbehältern die Rührreaktoren, Airliftreaktoren, Blasensäulen oder abwassertechnische Reaktoren wie z.B. Belebungsbecken oder Sequencing Batch Reaktoren darstellen.

9. Verfahren nach einem der vorherigen Ansprüche, **dadurch gekennzeichnet, dass** alle erforderlichen Vorkultivierungsstufen zur Erzeugung des Hefeinokulums steril und/oder unsteril erzeugt werden.

10. Verfahren nach einem der vorherigen Ansprüche, **dadurch gekennzeichnet, dass** die Kultivierung bei einem pH-Wert von 2,0 bis 8,0, vorzugsweise bei pH 3,5 bis 5,5, durchgeführt wird.

11. Verfahren nach einem der vorherigen Ansprüche, **dadurch gekennzeichnet, dass** die Kultivierung bei einer Temperatur von 5 bis 45°C, vorzugsweise bei 26 bis 33°C, durchgeführt wird.

12. Verfahren nach einem der vorherigen Ansprüche, **dadurch gekennzeichnet, dass** als Neutralisationsmittel CaCO₃, Ca(OH)₂, KOH, NaOH, Na₂CO₃, NaHCO₃, vorzugsweise NaOH, verwendet wird.

13. Verfahren nach einem der vorherigen Ansprüche, **dadurch gekennzeichnet, dass** die Gelöstsauerstoffkonzentration in einem Bereich von pO2 ≥1% bis 100%, vorzugsweise von pO2 ≥ 20%, während der Kultivierung eingestellt wird.

14. Verfahren nach einem der vorherigen Ansprüche, **dadurch gekennzeichnet, dass** die Kultivierung als Batch-, Fedbatch-, Repeated Fedbatch oder kontinuierlicher Prozess, vorzugsweise als Fedbatch- oder Repeated Fedbatch-Verfahren, durchgeführt wird.

15. Verfahren nach einem der vorherigen Ansprüche, **dadurch gekennzeichnet, dass** die Kultivierung ohne vorherige Immobilisierung der Mikroorganismen erfolgt.

## Claims

1. Method for the biotechnological production of carboxylic acids, in which microorganisms are used which are cultivated under non-sterile conditions in a cultivation medium which contains wastewater containing carbon-rich compounds and/or to which carbon-rich compounds are added, where
microorganisms strains of yeast species *Yarrowia lipolytica* are used;
carboxylic acids comprise aconitic, malic, succinic, citric, fumaric, isocitric, α-ketoglutaric, oxaloacetic, pyruvic and/or itaconic acids, preferably citric acid; and wherein
the carbon-rich compounds comprise mono-, di-, triglycerides, fatty acids, fatty acid methyl or ethyl esters, ethanol, methanol, glycerol, glucose, fructose, galactose, lactose, lactic acid, sucrose, xylose, arabinose and/or -nalkanes.

2. Method according to claim 1, **characterized in that** the strain *Yarrowia lipolytica* H181 (DSM 7806) is used.

3. Method according to claim 2, **characterized in that** the strain of *Yarrowia lipolytica* H181 (DSM 7806) is cultivated under non-sterile aerobic submerged conditions in a cultivation medium containing wastewater.

4. Method according to any of the preceding claims, **characterized in that** the wastewater used is a water contaminated by use, preferably the wastewater is a grey water, industrial wastewater from the food industry, chemical, pharmaceutical and biotechnological industry, vegetable oil processing, natural gas and petroleum extraction and- processing and/or municipal wastewater and/or from the purification stages of a kitchen and/or catering facility, effluents of commercial oil/fat separators and/or the primary clarification stage of wastewater treatment plants for municipal wastewater.

5. Method according to one of the preceding claims, **characterized in that** the wastewater is fed to a reaction vessel in a volume fraction of 10-100% of the total cultivation medium; preferably with a fraction of 90-100%.

6. Method according to one of the preceding claims, **characterized in that** waste products of used vegetable and/or animal fats and/or oils, preferably used frit oil, containing the carbon-rich compounds are used as carbon-rich compounds, or that mixtures of carbon-rich compounds are used.

7. Method according to one of the preceding claims, **characterized in that** the cultivation is carried out non-sterile and no measures and devices are provided for producing and maintaining sterility and preventing foreign germs from entering the cultivation medium.

8. Method according to any one of the preceding claims, **characterized in that** the cultivation is carried out in a reaction facility which is of open or closed design or construction, preferably in reaction vessels which are stirred reactors, airlift reactors, bubble columns or wastewater engineering reactors such as aeration tanks or sequencing batch reactors.

9. Method according to any one of the preceding claims, **characterized in that** all the necessary precultivation steps for producing the yeast inoculum are produced sterile and/or non-sterile.

10. Method according to any one of the preceding claims, **characterized in that** the cultivation is carried out at a pH of 2.0 to 8.0, preferably at pH 3.5 to 5.5.

11. Method according to any one of the preceding claims, **characterized in that** the cultivation is carried out at a temperature of from 5 to 45°C, preferably from 26 to 33°C.

12. Method according to one of the previous claims, **characterized in that** CaCO₃, Ca(OH)₂, KOH, NaOH, Na₂CO₃, NaHCO₃, preferably NaOH, is used as neutralizing agent.

13. Method according to any one of the preceding claims, **characterized in that** the dissolved oxygen concentration is adjusted in a range from pO2 ≥1% to 100%, preferably from pO2 ≥ 20%, during cultivation.

14. Method according to any one of the preceding claims, **characterized in that** the cultivation is carried out as a batch, fedbatch, repeated fedbatch or continuous process, preferably as a fedbatch or repeated fedbatch process.

15. Method according to any one of the preceding claims, **characterized in that** the cultivation is carried out without prior immobilization of the microorganisms.

## Revendications

1. Procédé de production biotechnologique d'acides carboxyliques, dans lequel on utilise des micro-organismes qui sont cultivés dans des conditions non stériles dans un milieu de culture qui contient des eaux usées qui contiennent des composés riches en carbone et/ou auquel on ajoute des composés riches en carbone, dans lequel
des souches de micro-organismes de l'espèce de *levure Yarrowia lipolytica* sont utilisés ; les acides carboxyliques comprennent l'acide aconitique, l'acide malique, l'acide succinique, l'acide citrique, l'acide fumarique, l'acide isocitrique, l'acide α-cétoglutarique, l'acide oxaloacétique, l'acide pyruvique et/ou l'acide itaconique, de préférence l'acide citrique ; et dans lequel
les composés riches en carbone comprennent des mono-, di-, triglycérides, acides gras, esters méthyliques ou éthyliques d'acides gras, éthanol, méthanol, glycérol, glucose, fructose, galactose, lactose, acide lactique, saccharose, xylose, arabinose et/ou -n-alcanes.

2. Procédé selon la revendication 1, **caractérisé en ce que** l'on utilise la souche *Yarrowia lipolytica* H181 (DSM 7806).

3. Procédé selon la revendication 2, **caractérisé en ce que** la souche de *Yarrowia lipolytica* H181 (DSM 7806) est cultivée dans des conditions aérobes submergées non stériles dans un milieu de culture contenant des eaux usées.

4. Procédé selon l'une des revendications précédentes, **caractérisé en ce que** les eaux usées utilisées sont des eaux contaminées par l'utilisation et l'usage, de préférence les eaux usées sont des eaux grises, des eaux usées industrielles provenant de l'industrie alimentaire, de l'industrie chimique, pharmaceutique et biotechnologique, du traitement d'huiles végétales, de l'extraction de gaz naturel et de pétrole et traitement et/ou des eaux usées communales et/ou des étapes de nettoyage d'une entreprise de cuisine et/ou de restauration, des effluents de séparateurs d'huile/de graisse industriels et/ou de l'étape de clarification primaire des installations de traitement des eaux usées communales.

5. Procédé selon l'une quelconque des revendications précédentes, **caractérisé en ce que** les eaux usées sont fournies à une cuve de réaction dans une proportion volumique de 10 à 100 % du milieu de culture total ; de préférence dans une proportion de 90 à 100 %.

6. Procédé selon l'une des revendications précédentes, **caractérisé en ce que** l'on utilise comme composés riches en carbone des déchets de graisses et/ou d'huiles végétales et/ou animales usagées, de préférence d'huile de friture usagée, qui contiennent les composés riches en carbone, ou **en ce que** l'on utilise des mélanges de composés riches en carbone.

7. Procédé selon l'une quelconque des revendications précédentes, **caractérisé en ce que** la culture est effectuée de manière non stérile et qu'aucune mesure ni aucun dispositif n'est prévu pour produire et maintenir une asepsie et une stérilité et pour éviter que des germes étrangers ne pénètrent dans le milieu de culture.

8. Procédé selon l'une quelconque des revendications précédentes, **caractérisé en ce que** la culture est réalisée dans un dispositif de réaction qui est de type ou de construction ouvert ou fermé, de préférence dans des cuves de réaction qui sont des réacteurs d'agitation, des réacteurs d'airlift, des colonnes à bulles ou des réacteurs de technique des eaux usées tels que des bassins d'aération ou des réacteurs de séquençage par lots.

9. Procédé selon l'une quelconque des revendications précédentes, **caractérisé en ce que** toutes les étapes de préculture nécessaires pour produire l'inoculum de levure sont produites de manière stérile et/ou non stérile.

10. Procédé selon l'une quelconque des revendications précédentes, **caractérisé en ce que** la culture est effectuée à un pH de 2,0 à 8,0, de préférence à un pH de 3,5 à 5,5.

11. Procédé selon l'une quelconque des revendications précédentes, **caractérisé en ce que** la culture est réalisée à une température de 5 à 45°C, de préférence de 26 à 33°C.

12. Procédé selon l'une des revendications précédentes, **caractérisé en ce que** l'on utilise comme agent de neutralisation CaCO₃, Ca(OH)₂, KOH, NaOH, Na₂CO₃, NaHCO₃, de préférence NaOH.

13. Procédé selon l'une quelconque des revendications précédentes, **caractérisé en ce que** la concentration en oxygène dissous est ajustée dans une plage de pO2 ≥1% à 100%, de préférence de pO2 ≥ 20%, pendant la culture.

14. Procédé selon l'une quelconque des revendications précédentes, **caractérisé en ce que** la culture est réalisée sous la forme d'un procédé de batch, d'un procédé de fed-batch, d'un procédé de fed-batch répété ou d'un procédé continu, de préférence un procédé de fed-batch ou un procédé de fed-batch répété.

15. Procédé selon l'une quelconque des revendications précédentes, **caractérisé en ce que** la culture est effectuée sans immobilisation préalable des micro-organismes.
